(19) Europäisches Patentamt
European Patent Office
Office européen des brevets

(11) EP 4 782 496 A1

# (12) EUROPEAN PATENT APPLICATION
published in accordance with Art. 153(4) EPC

(43) Date of publication:
**29.07.2026 Bulletin 2026/31**

(21) Application number: **24889152.5**

(22) Date of filing: **07.11.2024**

(51) International Patent Classification (IPC):
***C08K 5/098*** (2006.01) ***C08K 5/1545*** (2006.01)
***C08K 3/30*** (2006.01) ***C08L 1/28*** (2006.01)
***C08L 5/00*** (2006.01) ***C08B 15/00*** (2006.01)
***C08B 37/00*** (2006.01) ***C08J 3/12*** (2006.01)
***A61L 15/60*** (2006.01)

(52) Cooperative Patent Classification (CPC):
**A61L 15/60; C08B 15/00; C08B 37/00; C08J 3/12; C08K 3/30; C08K 5/098; C08K 5/1545; C08L 1/28; C08L 5/00**

(86) International application number:
**PCT/KR2024/017525**

(87) International publication number:
**WO 2025/100966 (15.05.2025 Gazette 2025/20)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **09.11.2023 KR 20230154734**

(71) Applicant: **LG Chem, Ltd.**
**Seoul 07336 (KR)**

(72) Inventors:
- **KANG, Sun Ah**
  **Daejeon 34122 (KR)**
- **CHO, Beom Shin**
  **Daejeon 34122 (KR)**
- **KIM, Yu Min**
  **Daejeon 34122 (KR)**
- **LEE, Dae Woong**
  **Daejeon 34122 (KR)**
- **KIM, Yeon Soo**
  **Daejeon 34122 (KR)**
- **YUN, Hae Sung**
  **Daejeon 34122 (KR)**

(74) Representative: **Plasseraud IP**
**104 Rue de Richelieu**
**CS92104**
**75080 Paris Cedex 02 (FR)**

## (54) POLYMER COMPOSITION

(57) The present specification discloses a polymer composition and a use thereof.

The present specification discloses a polymer composition prepared from materials having biodegradability, and exhibiting balanced absorption characteristics while exhibiting improved gel strength.

The present specification also discloses a method for preparing such a polymer composition and a use thereof.

EP 4 782 496 A1

## Description

### Technical Field

<u>Cross-citation with related applications</u>

This application claims the benefit of priority based on Korean Patent Application No. 10-2023-0154734 dated November 9, 2023, the disclosure of which is incorporated herein by reference in its entirety.

<u>Technical field</u>

The present specification discloses a polymer composition and a method for preparing the polymer composition.

### Background Art

A hydrogel polymer or hydrogel is generally defined as a cross-linked hydrophilic polymer. Such a polymer can be used as a material called an SAP (Super Absorbent Polymer).

The SAP is a material that can absorb moisture tens to thousands of times its own weight. The SAP is used for various applications, such as sanitary products such as hygiene products or diapers, medical products, household materials, agricultural materials, horticultural materials, transportation materials, civil engineering and construction materials, materials related to electrical and electronic devices, or water treatment agents. Among them, the SAP is often used as sanitary products such as diapers and sanitary pads, and in this case, it is required that it must have excellent absorption capacity for moisture, and the like, must not allow absorbed moisture to escape even under external pressures, and maintains its shape well even in a state swollen by absorbing water, thereby exhibiting excellent saline flow conductivity.

To exert excellent absorption characteristics and saline flow conductivity, it is important to exhibit high gel strength. However, since absorption characteristics generally decrease when trying to increase the gel strength of SAP, researches for securing excellent gel strength while maintaining absorption characteristics as much as possible are continued.

Among them, there is a method of adding insoluble inorganic fine particles or cationic polymer compounds as additives to surface-cross-linked absorbent materials. However, even with the above method, the gel strength was not sufficiently improved, and there was a problem that absorption capacity under pressure was reduced although the saline flow conductivity was improved, and there was a problem that the saline flow conductivity was reduced while the insoluble inorganic fine particles or cationic polymer compounds were separated from the surface of the superabsorbent particles over time.

In addition, even if the insoluble inorganic fine particles or cationic polymer compounds were introduced in the cross-linking step, there was a problem that the absorption characteristics and/or gel strength were not secured when the solvent was removed after the cross-linking process without performing a solvent removal process before the cross-linking process.

Furthermore, there is an attempt to produce the SAP using a polysaccharide-based natural polymer as a main raw material, to simultaneously secure excellent biodegradability degrees and absorption characteristics. For example, a method for producing the SAP using a polysaccharide component to which an acidic group is introduced is known. However, there was a problem that the absorption characteristics and/or gel strength were not secured because the cross-linking structure of the polysaccharide component changed depending on the degree of introduction of the acidic group.

Therefore, it is a difficult problem to obtain a material securing the gel strength in a certain level or more while maintaining the absorption characteristics.

### Disclosure

### Technical Problem

The present specification discloses a polymer composition and a method for preparing the same.

The present specification discloses a polymer composition prepared from materials having biodegradability, and exhibiting excellent absorption characteristics while exhibiting improved gel strength.

### Technical Solution

Among the physical properties mentioned in this specification, when the measurement temperature and/or

pressure affects the physical property value, the relevant physical property means a physical property measured at room temperature and/or normal pressure, unless specifically mentioned otherwise.

In the present specification, the term room temperature is a natural temperature without heating or cooling, which may mean, for example, any one temperature in a range of about 10°C to 30°C, or a temperature of 23°C or 25°C or so.

In the present specification, the term normal pressure is a pressure when not particularly reduced or increased, which may mean a pressure of atmospheric pressure or so, for example, about 740 mmHg to 780 mmHg or so.

Among the physical properties mentioned in this specification, when the measurement humidity affects the physical property value, the physical property means a physical property measured at natural humidity without special adjustment at the measured temperature and pressure, unless otherwise specified.

In this specification, unless otherwise specified, the term alkyl or alkyl group means an alkyl or alkyl group with 1 to 20 carbon atoms, 1 to 16 carbon atoms, 1 to 12 carbon atoms, 1 to 8 carbon atoms, or 1 to 4 carbon atoms. Such an alkyl or alkyl group may be linear, branched, or cyclic. Such an alkyl or alkyl group may also be optionally substituted with one or more substituents.

In this specification, unless otherwise specified, the term alkylene or alkylene group means a functional group which is connected to another object by separating two hydrogen atoms from an alkane, wherein it has a structure that the two hydrogen atoms are separated from other carbon atoms of the alkane. Such an alkylene or alkylene group may be an alkylene or alkylene group with 2 to 20 carbon atoms, 2 to 16 carbon atoms, 2 to 12 carbon atoms, 2 to 8 carbon atoms, or 2 to 4 carbon atoms. Such an alkylene or alkylene group may be linear, branched, or cyclic. Such an alkylene or alkylene group may also be optionally substituted with one or more substituents.

In this specification, unless otherwise specified, the term alkylidene or alkylidene group means a functional group which is connected to another object by separating two hydrogen atoms from an alkane, wherein it has a structure that the two hydrogen atoms are separated from one carbon atom of the alkane. Such an alkylidene or alkylidene group may be an alkylidene or alkylidene group with 1 to 20 carbon atoms, 1 to 16 carbon atoms, 1 to 12 carbon atoms, 1 to 8 carbon atoms, or 1 to 4 carbon atoms. Such an alkylidene or alkylidene group may be linear, branched, or cyclic. Such an alkylidene or alkylidene group may also be optionally substituted with one or more substituents.

The present specification discloses a polymer composition.

The term polymer composition means an absorbent material comprising polymers. The polymer composition may be a mixture of two or more different polymers. In the polymer composition, the two or more polymers may be simply mixed, or may also be physically or chemically bonded in whole or in part. The polymer may mean a relatively high molecular weight compound formed by connecting two or more unitary bodies by covalent bonds.

The polymer composition of the present specification may exhibit excellent centrifuge retention capacity (CRC) and absorption capacity under pressure (AUP) while exhibiting an appropriate level of gel strength.

For example, the lower limit of the gel strength of the polymer composition may be 2,500 Pa, 3,000 Pa, 4,000 Pa, 5,000 Pa, 6,000 Pa, 6,500 Pa, 7,000 Pa, 7,500 Pa, or 8,000 Pa or so, and the upper limit thereof may be 20,000 Pa, 18,000 Pa, 17,000 Pa, 15,000 Pa, 13,000 Pa, 11,000 Pa, 9,000 Pa, 8,000 Pa, 7,000 Pa, 6,000 Pa, or 5,500 Pa or so. The gel strength may be within a range of more than or equal to, or more than any one of the above-described lower limits; or within a range of less than or equal to, or less than any one of the above-described upper limits while being more than or equal to, or more than any one of the above-described lower limits. The gel strength may be a value measured in the manner described in "4. Gel strength" of Example sections of this specification.

For example, the lower limit of the centrifuge retention capacity (CRC) of the polymer composition according to EDANA (European Disposables and Nonwovens Association) method WSP 241.3 may be 15 *g/g,* 17 *g/g,* 18 g/g, 19 g/g, 20 g/g, 21 g/g, 22 g/g, 23 *g/g,* 24 *g/g,* 25 g/g, 26 g/g, 27 g/g, 28 g/g, 29 g/g, or 30 g/g or so, and the upper limit thereof may be 60 *g/g,* 55 *g/g,* 50 *g/g,* 45 g/g, 40 *g/g,* 35 g/g, 30 g/g, 25 g/g, or 24 g/g or so. The centrifuge retention capacity (CRC) may be within a range of more than or equal to, or more than any one of the above-described lower limits; or within a range of less than or equal to, or less than any one of the above-described upper limits while being more than or equal to, or more than any one of the above-described lower limits. The centrifuge retention capacity (CRC) may be a value measured in the manner described in "1. Centrifuge retention capacity (CRC)" of Example sections of this specification.

For example, the lower limit of the absorption capacity under pressure (AUP) of the polymer composition at 0.7 psi according to EDANA (European Disposables and Nonwovens Association) method WSP 242.3 may be 13 g/g, 14 g/g, 15 g/g, 16.5 g/g, 17 g/g, 17.5 g/g, 18 g/g, 18.5 g/g, 19 g/g, 19.5 g/g, or 20 g/g or so, and the upper limit thereof may be 60 g/g, 55 g/g, 50 g/g, 45 g/g, 40 g/g, 35 g/g, 30 g/g, 25 g/g, 20 g/g, or 19 g/g or so. The above absorption capacity under pressure (AUP) may be within a range of more than or equal to, or more than any one of the above-described lower limits; or within a range of less than or equal to, or less than any one of the above-described upper limits while being more than or equal to, or more than any one of the above-described lower limits. The absorption capacity under pressure (AUP) may be a value measured in the manner described in "2. Absorption capacity under pressure (AUP)" of Example sections of the present specification.

The polymer composition may exhibit any one characteristic of the above-described centrifuge retention capacity and absorption capacity under pressure while exhibiting the above-described gel strength, or may exhibit the above-described centrifuge retention capacity and absorption capacity under pressure while exhibiting the above-described gel strength.

The polymer composition of the present specification is prepared using a biodegradable material as a main component, which may exhibit excellent biodegradability.

For example, the lower limit of the biodegradability degree of the polymer composition may be 75%, 76%, 78%, 80%, 81%, or 82% or so, and the upper limit thereof may be 100%, 98%, 96%, 94%, 92%, 90%, 88%, 86%, 84%, 82%, 81%, or 80% or so. The biodegradability degree may be within a range of more than or equal to, or more than any one of the above-described lower limits; or within a range of less than or equal to, or less than any one of the above-described upper limits while being more than or equal to, or more than any one of the above-described lower limits. The biodegradability degree may be a value measured in the manner described in "3. Measurement of biodegradability degree" of Example sections of this specification.

For example, the lower limit of the polymer content in the polymer composition may be 55 wt%, 60 wt%, 65 wt%, 70 wt%, 75 wt%, 80 wt%, 85 wt%, 90 wt%, 95 wt%, or 98 wt% or so, and the upper limit thereof may be 100 wt%, 98 wt%, 96 wt%, 94 wt%, 92 wt%, or 90 wt% or so. The content may be within a range of more than or equal to, or more than any one of the above-described lower limits; or within a range of less than or equal to, or less than any one of the above-described upper limits while being more than or equal to, or more than any one of the above-described lower limits.

The polymer composition may comprise a polysaccharide component. For example, the polymer composition may be in the form of polymer particles including a core and a surface layer, where the polysaccharide component may be included in the core of the polymer composition.

The term polysaccharide component means a polysaccharide or a mixture of polysaccharides. In the case of a mixture of polysaccharides, the mixture may be a mixture of one type of polysaccharide (i.e., when two or more molecules of the same type of polysaccharide are present) or a mixture of two or more types of polysaccharides. Here, two or more types of polysaccharides may also mean different types of polysaccharides, and may also include polysaccharides which have the same type, but different physical properties such as a molecular weight.

The term polysaccharide has a meaning known in the industry. The polysaccharide generally refers to a polymer molecule in which two or more unitary bodies are linked by covalent bonds. The covalent bond connecting the unitary bodies is usually a glycosidic bond. Typically, a structure in which two unitary bodies are linked by a covalent bond such as a glycosidic bond is called a disaccharide, where in this specification, in the polysaccharide, only polysaccharides may be included, or the disaccharides may also be included in the category of polysaccharides.

The unitary body forming the polysaccharide may be a biomolecule composed of carbon, hydrogen, and oxygen, or composed of carbon, hydrogen, oxygen, and nitrogen. In this specification, the term biomolecule is interpreted to have the meaning generally applied in the industry. Typically, as an example of the biomolecule in the industry, monosaccharides such as glucose, galactose, fructose or xylose, disaccharides such as sucrose, lactose, maltose or trehalose, polyols such as sorbitol or mannitol, oligosaccharides such as maltodextrin, dextrin, raffinose, stachyose or fructo-oligosaccharide and/or amino sugars such as glucosamine or N-acetal glucosamine, and the like are known, but the types of biomolecules in the present specification are not limited to the foregoing.

In the polymer composition, the polysaccharide component may be in a cross-linked state. Here, the cross-linking means a state where two or more molecules of polysaccharide are connected by one or more chemical bonds. The cross-linking may also be formed by a chemical substance, which is referred to as a so-called cross-linking agent, other than the polysaccharide, and may be formed by a reaction between functional groups included in the polysaccharide.

In the present specification, the polymer composition may comprise at least a polysaccharide component cross-linked by a chemical substance other than the polysaccharide among the above types of cross-linked polysaccharides.

In the present specification, the cross-linking of the polysaccharide may be performed using a so-called acidic polysaccharide. As is known, the acidic polysaccharide is a polysaccharide having an acidic group, where an example of the acidic group may include a carboxylic group, a phosphate group, a phosphite group, and/or a sulfuric ester group, or a salt thereof.

A substitution degree of the acidic polysaccharide or the acidic polysaccharide component may be adjusted for an appropriate cross-linking reaction. The substitution degree is an indicator indicating the amount of the acidic group present in the acidic polysaccharide or polysaccharide component, where such a substitution degree may be, for example, a value obtained before the polysaccharide or polysaccharide component implements the cross-linked structure.

For example, the lower limit of the substitution degree may be 0.1, 0.15, 0.2, 0.25, 0.3, 0.35, 0.4, 0.45, 0.5, 0.6, 0.65, 0.7, 0.75, 0.8, or 0.83 or so, and the upper limit thereof may be 2.5, 2, 1.5, 1.4, 1.3, 1.2, 1.1, 1.05, 1, 0.95, 0.9, or 0.85 or so. The substitution degree may be within a range of more than or equal to, or more than any one of the above-described lower limits; or within a range of less than or equal to, or less than any one of the above-described upper limits; or within a range of less than or equal to, or less than any one of the above-described upper limits while being more than or equal to, or more than any one of the above-described lower limits. The substitution degree may be a value measured in the manner described in "7. Evaluation of substitution degree" of Example sections of this specification.

Within the range of the substitution degree, the cross-linking of the polysaccharide may proceed effectively, and

the physical properties (for example, gel strength, absorption capacity and/or biodegradability, etc.) of the resulting polymer composition may be stably secured. When the substitution degree is high, the number of acidic groups, which are hydrophilic functional groups, increases in the polysaccharide or polysaccharide component, so that it may be advantageous in terms of absorption characteristics, but too many acidic groups excessively promote the cross-linking reaction, whereby absorption characteristics after cross-linking may be lowered. Therefore, considering these points, it is possible to select an appropriate substitution degree.

In this specification, the substitution degree is an indicator of the degree of acidic group present in the polysaccharide or polysaccharide component, which is, for example, a value representing to what extent the functional group such as a hydroxy group present in each unitary body contained in the polysaccharide or polysaccharide component is substituted with a predetermined acidic group (for example, the carboxyl group) and is an average value for each unitary body present in the polysaccharide. For example, if the unitary body is a grape sugar (glucose) unit, there are three hydroxy groups in the relevant unit before modification, and thus, if all the hydroxy groups are replaced with the functional groups of Formula 2, the substitution degree for the relevant unit is 3. However, the substitution degree of the polysaccharide is the average value of the substitution degree of each unitary body present in the relevant polysaccharide, so that for example, in a polysaccharide containing 5 grape sugar (glucose) units, if the substitution degree of each unit is 1, 0, 2, 3, and 1, the substitution degree of the polysaccharide becomes 1.4, which is the average value. This substitution degree may be identified through $^{1}H$ NMR analysis of the polysaccharide. That is, since the hydroxyl groups and substituted functional groups present in the polysaccharide can be quantified through the $^{1}H$ NMR analysis, the substitution degree can be identified, and the substitution degree can be calculated in consideration of the $^{1}H$ NMR analysis results of the polysaccharide before modification, if necessary. In this way, the method of quantifying functional groups through $^{1}H$ NMR analysis is known. In order that the binding of the functional group present in the polysaccharide component or the substituted functional group with the metal component, and the cross-linking with the cross-linkable functional group smoothly proceed, the substitution degree has an important meaning.

The polysaccharide having the carboxyl group is not particularly limited. For example, the cross-linking reaction may be performed by applying polysaccharides having carboxyl groups on their own, such as so-called CMC (carboxylmethyl cellulose), corresponding to a wood-based polysaccharide, or polysaccharides to which carboxyl groups are introduced through processes such as maleation or carboxyalkylation.

In one example, the polysaccharide component may contain a unit represented by Formula 1 below.

[Formula 1]

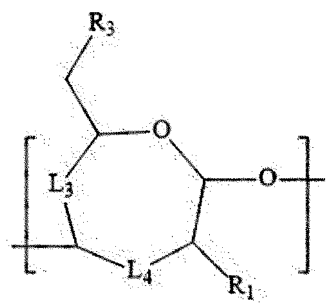

In Formula 1, $R_1$ is a hydroxy group, an amino group, $-L_5-C(=O)-OH$, $-L_5-C(=O)-O-$, or a functional group of Formula 2 below, $R_3$ is a hydroxy group, $-L_5-C(=O)-OH$, $-L_5-C(=O)-O-$, or a functional group of Formula 2 below, and any one of $L_3$ and $L_4$ is a single bond, and the other is $CHR_2$, where $R_2$ is a hydroxy group, $-L_5-C(=O)-OH$, $-L_5-C(=O)-O-$, or a functional group of Formula 2 below, and $L_5$ is an alkylene group or an alkylidene group, provided that an oxygen or nitrogen atom of any one of $R_1$ to $R_3$ above may form a bond with a metal component to be described below.

[Formula 2]

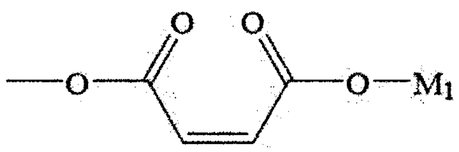

In Formula 2, $M_1$ is hydrogen or a metal, and when $M_1$ is the metal, the bond of $O-M_1$ is an ionic bond.

In Formula 1, the case where $R_1$ is an amino group is a case where the unit is a glucosamine unit or an N-acetylglucosamine unit. The amino group at this time may be optionally substituted with one or more substituents. The

substituent at this time may be exemplified by an alkyl group or an alkylcarbonyl group. In this case, the alkyl group may be an alkyl group with 1 to 20 carbon atoms, 1 to 16 carbon atoms, 1 to 12 carbon atoms, 1 to 8 carbon atoms, or 1 to 4 carbon atoms, or a methyl group, and such an alkyl group may be linear, branched, or cyclic, which may also be optionally substituted with one or more substituents.

The functional group of $-L_5-C(=O)-OH$ or $-L_5-C(=O)-O^-$ in Formula 1 may be, for example, a carboxyl group introduced by carboxyalkylation or a functional group in which the carboxyl group is ionized, and the like, and Formula 2 is a functional group introduced by maleation.

Such functional groups are introduced, to form cross-linking bonds by participating in the cross-linking reaction, but all the introduced functional groups may not participate in the cross-linking reaction, and in this case, some functional groups may also remain.

Here, the matter that any one of $L_3$ and $L_4$ is a single bond means that any one of $L_3$ and $L_4$ does not exist. For example, if $L_3$ does not exist, the carbon atoms connected to the left and right of $L_3$ in Formula 1 are directly connected, and if $L_4$ does not exist, the carbon atoms connected to the left and right of $L_4$ in Formula 1 are directly connected.

The matter that the other of $L_3$ and $L_4$ above is $CHR_2$ means a case where any one of $L_3$ and $L_4$ in Formula 3 is a carbon atom, and the carbon atom is substituted with a substituent $R_2$.

The ratio of the polysaccharide component in the polymer composition is not particularly limited, but the higher the ratio, the greater the biodegradability of the polymer material. However, in the case of absorbent materials applying conventional polysaccharide components, if the ratio of polysaccharide components is excessively increased in consideration of biodegradability, there is a problem that the absorption capacity is reduced. However, in the present specification, the desired absorption capacity can be stably achieved while maintaining the ratio of polysaccharide components at a high level.

For example, the lower limit of the ratio of the polysaccharide component in the polymer composition may be 85 wt%, 86 wt%, 87 wt%, 88 wt%, 89 wt%, 90 wt%, 91 wt%, 92 wt%, 93 wt%, 94 wt%, or 95 wt% or so, and the upper limit thereof may be 100 wt%, 98 wt%, 96 wt%, 94 wt%, 92 wt%, 90 wt%, 88 wt%, or 86 wt% or so. The ratio may be within a range of more than or equal to, or more than any one of the above-described lower limits; or within a range of less than or equal to, or less than any one of the above-described upper limits while being more than or equal to, or more than any one of the above-described lower limits.

The polymer composition of the present specification may comprise a metal component.

For example, the composition may comprise a polymer cross-linking the polysaccharide component with a metal compound. The polymer may comprise a metal component bound to the polysaccharide component. For example, the polymer may be included in the core of the polymer composition, and thus, the metal component may be included in the core of the polymer composition.

Here, the metal compound may exist as a metal salt in an aqueous solution, or may exist as a state of metal ions as the metal salt is decomposed, or may be a state where the metal ions are bound to a water molecule, or may also be a state where they exist together.

For example, an aluminum compound may be applied as the metal compound. As the aluminum compound, one or more selected from the group consisting of aluminum acetate, aluminum sulfate, aluminum hydrochloride, and aluminum lactate may be used.

In the present specification, the term metal component means a substance existing in a state where metal ions are bound to a polysaccharide component in the polymer composition. Such a metal component may be a component derived from the metal compound.

By applying a metal component to perform a cross-linking reaction of polysaccharides, the present specification can provide a novel polymer composition having excellent absorption capacity while having appropriate gel strength. In one example, when the metal component (metal ion) and the polysaccharide component in the core of the polymer composition exist in a state of being bonded to each other, the bond may be a coordination bond. In this case, it may be one formed with the metal ion by providing an electron pair from an oxygen atom or a nitrogen atom included in the functional group of the polysaccharide component.

Therefore, the core of the polymer composition of the present specification may comprise a polysaccharide component and a metal component, which are coordinately bonded to each other.

In one example, the metal component may form a bond with an oxygen atom or a nitrogen atom included in a functional group in one unitary body, or the metal component may also form a bond with an oxygen atom or a nitrogen atom included in a functional group in another unitary body while forming a bond with an oxygen atom or a nitrogen atom included in a functional group in one unitary body.

As the polymer composition of the present specification comprises the metal component, the mechanical properties of the polymer composition are improved due to the excellent strength of the metal itself, or by the strong network of the polysaccharide component through bonding with the polysaccharide component, whereby it can improve the gel strength.

In one example, the polymer composition may comprise the metal component in a certain content or more based

on the total weight of the polymer composition. For example, the lower limit of the content of the metal component may be 100 ppm, 150 ppm, 200 ppm, 250 ppm, 300 ppm, 350 ppm, 400 ppm, 450 ppm, or 500 ppm or so, and the upper limit thereof may be 1,000 ppm, 900 ppm, 800 ppm, 700 ppm, 600 ppm, 500 ppm, 400 ppm, 300 ppm, or 200 ppm or so. The metal component may be within a range of more than or equal to, or more than any one of the above-described lower limits; or within a range of less than or equal to, or less than any one of the above-described upper limits; or within a range of less than or equal to, or less than any one of the above-described upper limits while being more than or equal to, or more than any one of the above-described lower limits. The content of the metal component may be a content measured using inductively coupled plasma optical emission spectrometry (ICP-OES), which is a method described in "5. Metal component content" of Example sections of this specification, under conditions of RF power 1300 W, torch height 15 mm, plasma gas flow 15 L/min, sample gas flow 0.8 L/min, Aux. gas flow 0.2 L/min, and pump speed 1.5 mL/min. When the metal component is included within the content range, it is possible to secure excellent gel strength while maintaining absorption characteristics to the maximum extent.

The polymer composition may be in the form of a polymer particle including the core and the surface layer, and the surface layer may comprise a compound covalently bonded to the surface of the core. For example, the compound of the surface layer may be covalently bonded to the cross-linked polysaccharide component of the core.

The surface layer may be introduced by reacting the cross-linked polysaccharide component with the compound. For example, the core may be in a powder state formed through a pulverization process or the like, and the surface layer may be formed by reacting the core in the powder state with the compound.

That is, the polymer composition of the present specification may be provided in a state where two or more molecules of polysaccharide are cross-linked by a cross-linking reaction and then the compound is applied to further cross-link them. This further cross-linking is introduced for improving the gel strength of the cross-linked polysaccharide component and improving the absorption capacity under pressure (AUP) and/or saline flow conductivity. In this specification, it is possible to provide a polymer composition exhibiting balanced absorption capacity and gel strength even after application of the compound through control of the cross-linking conditions in the cross-linking step.

The compound may also be introduced, for example, by subjecting the cross-linked polysaccharide component to a treatment such as pulverization to powder it and reacting the surface of the relevant powder with the compound. That is, the compound may serve as a surface treatment agent for the core. In this case, the polymer composition may comprise the cross-linked polysaccharide component in the form of particles and the compound bound to the surface of the particle. In such a case, the size of the particles is not particularly limited, which may be controlled to an appropriate size depending on the applied use. Typically, the size of the particle may be within a range of approximately 100 $\mu$m to 1,000 $\mu$m.

As the compound, a substance having two or more functional groups capable of reacting with the functional group (hydroxy group, amino group, or carboxyl group, etc.) of the polysaccharide component may be used. The compound may have 2 to 10, 2 to 9, 2 to 8, 2 to 7, 2 to 6, 2 to 5, 2 to 4, or 2 to 3, or 2 or 3 functional groups capable of reacting with the functional group (hydroxy group, amino group, or carboxyl group, etc.) of the polysaccharide component.

As the type of the applicable compound, one or more selected from the group consisting of a polyfunctional epoxy compound, an epoxy silane compound, an amino silane compound, epichlorohydrin, an acyl chloride, a carbonate, a dialdehyde, a diamine, a diol, carbon disulfide, phosphoryl chloride, divinyl benzene, an organic acid, and an organic acid anhydride may be used.

For performing effective cross-linking and securing desired physical properties, it may be advantageous to use a specific type of compound as the compound.

In one example, as the compound, an oxidized disaccharide, or an oxidized polysaccharide may be applied. For example, a disaccharide having two or more aldehyde or carboxyl groups may be applied.

Such an oxidized disaccharide may be exemplified by oxidized maltose, oxidized lactose, oxidized sucrose, oxidized trehalose, and the like, but is not limited thereto. The oxidized disaccharide may be a disaccharide in which the hydroxyl groups of C-2 and C-3 are oxidized to aldehyde groups, and may also be a disaccharide in which the aldehyde group is oxidized to a carboxyl group, while the bond between C-2 and C-3 is broken by adding an oxidizing agent to the disaccharide. The polymer composition treated with the compound as above can satisfy the desired absorption characteristics and biodegradability degree while exhibiting appropriate gel strength.

The lower limit of the weight ratio of the compound relative to 100 parts by weight of the cross-linked polysaccharide component in the polymer composition may be 0.01 parts by weight, 0.05 parts by weight, 0.1 parts by weight, 0.5 parts by weight, 1 part by weight, 1.5 parts by weight, or 2 parts by weight or so, and the upper limit thereof may be 10 parts by weight, 8 parts by weight, 6 parts by weight, 4 parts by weight, or 2 parts by weight or so. The ratio may be within a range of more than or equal to, or more than any one of the above-described lower limits; or within a range of less than or equal to, or less than any one of the above-described upper limits; or within a range of less than or equal to, or less than any one of the above-described upper limits while being more than or equal to, or more than any one of the above-described lower limits. Under the ratio as above, the polymer composition can satisfy the desired absorption characteristics and biodegradability degree while exhibiting appropriate gel strength.

The polymer composition may comprise the polysaccharide component (the polysaccharide component cross-linked through the metal component and bonded to the compound) as described above, and may further comprise other components if necessary.

The present specification discloses a method for preparing such a polymer composition.

The preparing method may comprise a step of cross-linking a polysaccharide component with a metal compound. For example, the cross-linking may be performed by using a polymer solution comprising a polysaccharide component, a metal compound, and a solvent to cross-link the polysaccharide component in the solution with the metal compound.

As the cross-linked polysaccharide component, the above-described acidic polysaccharide (for example, the acidic polysaccharide having the substitution degree) may be used.

For performing effective cross-linking and securing excellent gel strength, it may be advantageous to use the metal compound in a specific content. For example, the lower limit of the weight ratio of the metal compound in the polymer relative to 100 parts by weight of the polysaccharide component may be 0.01 parts by weight, 0.05 parts by weight, 0.1 parts by weight, 0.2 parts by weight, 0.3 parts by weight, 0.4 parts by weight, or 0.5 parts by weight or so, and the upper limit thereof may be 10 parts by weight, 8 parts by weight, 6 parts by weight, 4 parts by weight, 2 parts by weight, 1 part by weight, 0.9 parts by weight, 0.8 parts by weight, 0.7 parts by weight, 0.6 parts by weight, 0.5 parts by weight, 0.4 parts by weight, 0.3 parts by weight, or 0.2 parts by weight or so. The ratio may be within a range of more than or equal to, or more than any one of the above-described lower limits; or within a range of less than or equal to, or less than any one of the above-described upper limits; or within a range of less than or equal to, or less than any one of the above-described upper limits while being more than or equal to, or more than any one of the above-described lower limits. Under the ratio as above, the polymer composition can satisfy the desired absorption characteristics and biodegradability degree while exhibiting appropriate gel strength.

For performing effective cross-linking and securing excellent gel strength, the pH of the polymer solution upon the cross-linking reaction may be adjusted and/or maintained within a predetermined range. For example, the lower limit of the pH of the polymer solution may be 6, 6.5, or 7 or so, and the upper limit thereof may be 10, 9, 8, 7.5, 7, or 6.5 or so. The pH may be within a range of less than or equal to, or less than any one of the above-described upper limits; or within a range of more than or equal to, or more than any one of the above-described lower limits; or within a range of less than or equal to, or less than any one of the above-described upper limits while being more than or equal to, or more than any one of the above-described lower limits.

The reason why the desired resultant can be obtained through cross-linking in the pH range is not clear, but it is expected that the pH affects the cross-linking rate of the polysaccharide or the cross-linking density of the cross-linked polysaccharide component, and this result is strengthened in the reaction with the metal component, so that a cross-linking structure capable of exerting excellent absorption capacity while exhibiting appropriate gel strength is implemented.

In the above process, an aqueous solvent, such as water, may be used as the solvent, and specifically, tap water, distilled water, deionized water, or purified water may be used. It may be appropriate to substantially use only the aqueous solvent (e.g., water) as the solvent upon cross-linking. Therefore, the solvent used upon the cross-linking may not substantially include any other solvent other than the aqueous solvent (e.g., water). At this time, the matter of substantially not including another solvent may mean a case where the upper limit of the content of another solvent other than the aqueous solvent (e.g., water) in the solvent is 10 wt%, 9 wt%, 8 wt%, 7 wt%, 6 wt%, 5 wt%, 4 wt%, 3 wt%, 2 wt%, 1 wt%, 0.5 wt%, 0.1 wt%, 0.05 wt%, 0.01 wt%, 0.005 wt%, or 0.001 wt% or so, and the lower limit thereof is 0 wt% or so. The ratio may be within a range of less than or equal to, or less than any one of the above-described upper limits; or within a range of less than or equal to, or less than any one of the above-described upper limits while being more than or equal to, or more than any one of the above-described lower limits.

The amount of the solvent applied in the above process may be an amount of 8 to 100 times, 8 to 80 times, 8 to 60 times, 8 to 50 times, 10 to 50 times, 20 to 50 times, 30 to 50 times, or 35 to 45 times the weight of the applied polysaccharide. The dissolution of the polysaccharide into the solvent may be carried out under room temperature and normal pressure conditions, but is not limited thereto.

The cross-linking may proceed by dissolving the polysaccharide in the solvent and maintaining the pH within the above-described range. If necessary, additional processes, such as a stirring process, capable of promoting the cross-linking may also be performed. The method of maintaining the pH within the above range is not particularly limited, and if the pH within the above range is achieved by itself by adding the polysaccharide and/or the cross-linking agent, the cross-linking may proceed in that state, and if the desired pH is not achieved, the pH may be adjusted by adding an appropriate acid or base in consideration of the desired pH. At this time, as the base, for example, the hydroxide applied in the carboxyalkylation may be used, and as the acid, hydrochloric acid or sulfuric acid may be used, without being limited thereto.

In the above reaction process, a catalyst may be added as needed. For example, an ester catalyst promoting the reaction of carboxyl groups and hydroxy groups may be added. As such a catalyst, 4-methylaminopyridine, magnesium acetate, tetra-n-butyl titanate, lead acetate, sodium acetate, potassium acetate, antimony trioxide and/or N-methylimidazole, and the like may be used, without being limited thereto. The catalyst may be added in a catalytic amount and, for

example, may be used in a ratio within a range of 0.1 mol to 5 mol relative to 1 mol of the polysaccharide applied to the reaction. The lower limit of the catalyst usage ratio may be 0.1 mol, 0.5 mol, 1 mol, or 2 mol or so, and the upper limit thereof may be 5 mol, 4.5 mol, 4 mol, or 3.5 mol or so. The ratio may be within a range of less than or equal to, or less than any one of the above-described upper limits; or within a range of less than or equal to, or less than any one of the above-described upper limits while being more than or equal to, or more than any one of the above-described lower limits.

The reaction may also be performed in the presence of a heat stabilizer, if necessary. As the applicable heat stabilizer, an organic or inorganic phosphorus compound such as phosphoric acid, an organic ester of phosphoric acid, phosphorous acid or an organic ester of phosphorous acid may be used, and for example, phosphoric acid, alkyl phosphate or aryl phosphate, and the like, which is commercially known as the heat stabilizer, may be used.

The reaction may also be performed in the presence of additives such as thickeners, plasticizers, preservation stabilizers, and/or antioxidants, if necessary.

In one example, the process of obtaining the cross-linked polysaccharide component may comprise a first step of maintaining the polymer solution at a temperature T1; and a second step of maintaining the polymer solution at a temperature T2.

For example, the temperature T1 of the first step may be adjusted so that ΔT1 of the Equation 1 below is within a range of 10°C to 90°C.

[Equation 1]

$$\Delta T1 = Tb - T1$$

In Equation 1, Tb is the boiling point of the solvent of the polymer solution under 1 atm, and T1 is the temperature T1 of the first step.

For example, the lower limit of Tb in Equation 1 above may be 60°C, 70°C, 80°C, 85°C, 90°C, 95°C, or 100°C or so, and the upper limit thereof may be 200°C, 180°C, 160°C, 140°C, 120°C, 110°C, or 100°C or so. The Tb may be within a range of more than or equal to, or more than any one of the above-described lower limits; or within a range of less than or equal to, or less than any one of the above-described upper limits; or within a range of less than or equal to, or less than any one of the above-described upper limits while being more than or equal to, or more than any one of the above-described lower limits.

For example, the lower limit of T1 in Equation 1 above may be 30°C, 35°C, 36°C, 38°C, or 40°C or so, and the upper limit thereof may be 200°C, 150°C, 120°C, 100°C, 80°C, 60°C, 50°C, or 40°C or so. The T1 may be within a range of more than or equal to, or more than any one of the above-described lower limits; or within a range of less than or equal to, or less than any one of the above-described upper limits; or within a range of less than or equal to, or less than any one of the above-described upper limits while being more than or equal to, or more than any one of the above-described lower limits.

For example, the lower limit of ΔT1 in Equation 1 above may be 10°C, 20°C, 30°C, 40°C, 50°C, or 60°C or so, and the upper limit thereof may be 90°C, 80°C, 70°C, or 60°C or so. The ΔT1 may be within a range of more than or equal to, or more than any one of the above-described lower limits; or within a range of less than or equal to, or less than any one of the above-described upper limits; or within a range of less than or equal to, or less than any one of the above-described upper limits while being more than or equal to, or more than any one of the above-described lower limits.

For example, the temperature T2 of the second step may be adjusted so that ΔT2 of Equation 2 below is within a range of 5°C to 200°C.

[Equation 2]

$$\Delta T2 = T2 - Tb$$

In Equation 2, Tb is the boiling point of the solvent of the polymer solution under 1 atm, and T2 is the temperature T2 of the second step.

For example, the lower limit of Tb in Equation 2 above may be 60°C, 70°C, 80°C, 85°C, 90°C, 95°C, or 100°C or so, and the upper limit thereof may be 200°C, 180°C, 160°C, 140°C, 120°C, 110°C, or 100°C or so. The Tb may be within a range of more than or equal to, or more than any one of the above-described lower limits; or within a range of less than or equal to, or less than any one of the above-described upper limits; or within a range of less than or equal to, or less than any one of the above-described upper limits while being more than or equal to, or more than any one of the above-described lower limits.

For example, the lower limit of T2 in Equation 2 above may be 80°C, 85°C, 90°C, 95°C, 100°C, 105°C, 110°C,

115°C, or 120°C or so, and the upper limit thereof may be 300°C, 250°C, 240°C, 220°C, 200°C, 180°C, 160°C, 140°C, 130°C, or 120°C or so. The T2 may be within a range of more than or equal to, or more than any one of the above-described lower limits; or within a range of less than or equal to, or less than any one of the above-described upper limits; or within a range of less than or equal to, or less than any one of the above-described upper limits while being more than or equal to, or more than any one of the above-described lower limits. Such a temperature may be secured by using, for example, a method, such as hot air supply, infrared irradiation, microwave irradiation, or ultraviolet irradiation.

For example, the lower limit of ΔT2 in Equation 2 above may be 5°C, 10°C, 15°C, or 20°C or so, and the upper limit thereof may be 200°C, 150°C, 100°C, 80°C, 60°C, 40°C, or 20°C or so. The ΔT2 may be within a range of more than or equal to, or more than any one of the above-described lower limits; or within a range of less than or equal to, or less than any one of the above-described upper limits; or within a range of less than or equal to, or less than any one of the above-described upper limits while being more than or equal to, or more than any one of the above-described lower limits.

The time during which the first step is performed is not particularly limited, and for example, the lower limit of the time may be 10 hours, 15 hours, 20 hours, 21 hours, 22 hours, 23 hours, or 24 hours or so, and the upper limit thereof may be 60 hours, 50 hours, 48 hours, 40 hours, 35 hours, 30 hours, 28 hours, 26 hours, or 24 hours or so. The time may be within a range of more than or equal to, or more than any one of the above-described lower limits; or within a range of less than or equal to, or less than any one of the above-described upper limits; or within a range of less than or equal to, or less than any one of the above-described upper limits while being more than or equal to, or more than any one of the above-described lower limits.

The time for which the second step is performed is not particularly limited, and for example, the lower limit of the time may be 10 minutes, 15 minutes, 20 minutes, 25 minutes, or 30 minutes or so, and the upper limit thereof may be 10 hours, 5 hours, 3 hours, 2 hours, 1 hour, 50 minutes, 40 minutes, or 30 minutes or so. The time may be within a range of more than or equal to, or more than any one of the above-described lower limits; or within a range of less than or equal to, or less than any one of the above-described upper limits; or within a range of less than or equal to, or less than any one of the above-described upper limits while being more than or equal to, or more than any one of the above-described lower limits.

The cross-linked polysaccharide component obtained through the above step may react with the above-described additional compound in a subsequent reaction. After the cross-linking process, the polysaccharide component may also react with the compound as such, or may react with the compound after additional treatment, to increase the cross-linking efficiency, if necessary. For example, after the cross-linking process, the polysaccharide component may be granulated and then reacted with the compound. For example, the polysaccharide component may be granulated through an appropriate grinding and/or classification process, and the granulated polysaccharide component may be reacted with the compound.

In this case, the process may comprise a step of granulating the cross-linked polysaccharide component, which is the cross-linked product as cross-linked; and a step of reacting the granulated cross-linked polysaccharide component with the compound.

The method of reacting the polysaccharide component with the compound is not particularly limited. For example, a method of contacting the polysaccharide component with the compound in an appropriate reaction medium, such as a solvent, and reacting them may be applied. In the case where the granulated polysaccharide component and the compound react, a method of contacting the compound or a cross-linking solution containing the compound by spraying it on the surface of the granulated polysaccharide component and proceeding with the reaction may be used.

The reaction with the compound may be performed in the presence of additives, such as catalysts, thickeners, plasticizers, preservation stabilizers, and/or antioxidants, if necessary.

The reaction with the compound may be performed at a predetermined temperature. For example, the lower limit of the temperature at which the reaction proceeds may be 40°C, 50°C, 60°C, 70°C, 80°C, 85°C, 90°C, 95°C, 100°C, 105°C, 110°C, 115°C, or 120°C or so, and the upper limit thereof may be 300°C, 280°C, 260°C, 240°C, 220°C, 200°C, 180°C, 160°C, 140°C, 130°C, 125°C, or 120°C or so. The temperature may be within a range of less than or equal to, or less than any one of the above-described upper limits; or within a range of less than or equal to, or less than any one of the above-described upper limits while being more than or equal to, or more than any one of the above-described lower limits. Such a reaction temperature may be achieved by a method, such as hot air supply, infrared irradiation, microwave irradiation, or ultraviolet irradiation.

The time for the reaction to proceed is not particularly limited, and for example, the lower limit of the reaction time may be 10 minutes, 20 minutes, or 30 minutes or so, and the upper limit thereof may be 10 hours, 8 hours, 6 hours, 4 hours, 2 hours, 60 minutes, 40 minutes, or 35 minutes or so. The time may be within a range of less than or equal to, or less than any one of the above-described upper limits; or within a range of less than or equal to, or less than any one of the above-described upper limits while being more than or equal to, or more than any one of the above-described lower limits.

Following the reaction with such a compound, the desired polymer composition may be obtained through an additional process (for example, a grinding/classifying process, etc.), if necessary.

The polymer composition is made of a biodegradable material and exhibits balanced absorbency, so that it may be used for various applications.

For example, the polymer composition may be used as sanitary products such as diapers or sanitary napkins, or absorbent materials applied to other applications requiring absorption. If necessary, further cross-linking, surface treatment, or physical grinding processes may also be performed on the polymer composition to increase the efficiency for use as the sanitary products or absorbent materials.

Therefore, the present specification discloses an absorbent material or sanitary product (e.g., diapers, sanitary napkins, etc.) comprising the polymer composition.

The specific method of forming the absorbent material or sanitary product by applying the polymer composition is not particularly limited, and for example, the method of forming the absorbent material or sanitary product by applying the existing SAP may be used in the same manner.

**Advantageous Effects**

The present specification discloses a polymer composition and a method for preparing the same.

The present specification discloses a polymer composition made of a material having biodegradability and exhibiting improved gel strength while exhibiting excellent absorption characteristics.

**Mode for Invention**

Hereinafter, the polymer composition and the like will be specifically described with reference to examples, but the scope of the polymer composition and the like is not limited by the following examples.

**1. Centrifuge retention capacity (CRC)**

Centrifuge retention capacity (CRC) was measured according to EDANA (European Disposables and Nonwovens Association) WSP 241.3. About 0.2 g ($W_0$) of the obtained polymer particles was placed in a non-woven bag, sealed, and then submerged in a physiological saline solution. As the physiological saline solution, an aqueous NaCl solution with a concentration of 0.9 wt% was used. The state was maintained for 30 minutes or so, water was removed from the bag for 3 minutes under a condition of 250 G using a centrifuge, and the mass (g, $W_2$) of the bag was measured. The same operation was performed on the same non-woven bag containing no polymer particle, and the mass (g, $W_1$) was measured. The CRC (g/g) was calculated by substituting the measurement results into Equation A below. The evaluation was conducted under constant temperature and humidity conditions (23 ± 1°C, relative humidity: 50 ± 10%).

[Equation A]

$$\mathrm{CRC}\ (g/g) = \{[W_2(g) - W_1(g)]/W_0(g)\} - 1$$

**2. Absorption capacity under pressure (AUP)**

Absorption capacity under pressure (AUP, 0.7 psi) of polymer particles was measured according to the standard of EDANA (European Disposables and Nonwovens Association) WSP 242.3. A 400-mesh stainless steel wire net was mounted on the bottom of a plastic cylinder with an inner diameter of 60 mm or so. Under conditions of a temperature of 23 ± 2°C and a relative humidity of 50%, about 0.90 g ($W_0$) of the polymer particles was uniformly sprayed on the wire net, and a piston capable of further uniformly imparting a load of about 0.7 psi was installed thereon to manufacture a measuring device. As the piston, a piston with an outer diameter slightly smaller than 60 mm was used, which was installed so that it could move up and down without forming a gap with the inner wall of the cylinder. The weight (unit: g) ($W_3$) of the measuring device was measured. A glass filter with a diameter of 90 mm or so and a thickness of 5 mm or so was placed on the inside of a petro dish with a diameter of about 150 mm, and a physiological saline solution (NaCl aqueous solution with a concentration of 0.9 wt%) was applied to be at the same level as the upper surface of the glass filter. One sheet of filter paper with a diameter of 90 mm or so was placed thereon. The measuring device was placed on the filter paper and the physiological saline solution was absorbed for 1 hour under a load of 0.7 psi. After 1 hour, the measuring device was lifted, and the weight $W_4$ (g) was measured. The absorption capacity under pressure (AUP) (g/g) was calculated by substituting the respective weights as measured into Equation B below.

[Formula B]

$$AUP\ (g/g) = [W_4(g) - W_3(g)]/W_0(g)$$

**3. Measurement of biodegradability degree**

A biodegradability degree was measured in the manner specified in ISO 14855-1 (2005) standard. The above standard is a method of measuring an aerobic biodegradability degree of plastic materials under a composting condition, which is a method that the biodegradability degree of polymer particles is calculated by quantifying the amount of carbon dioxide emitted by microorganisms metabolizing the relevant material. As the polymer particles were applied to the composting condition according to the standard, the biodegradability degree was measured for 6 months, and the biodegradability degree was obtained as the ratio of the theoretical carbon dioxide generation amount and the actual carbon dioxide generation amount of the material. Here, the theoretical carbon dioxide generation amount and biodegradability degree are obtained according to the following equations C and D, respectively.

[Equation C]

$$\text{Theoretical carbon dioxide generation amount } (ThCO_2, \text{ g/container}) = M_{TOT} \times C_{TOT} \times (44/12)$$

In Equation C, $M_{TOT}$ is the amount (g) of total dry solid content in the test material (polymer particles) added to the compost at the start of the measurement, and $C_{TOT}$ means the ratio (g/g) of organic carbon contained in the total dry solid content of the test material.

[Equation D]

$$\text{Biodegradability degree (\%)} = [\{(CO_2)_T - (CO_2)_B\}/ThCO_2] \times 100$$

In Equation D, $(CO_2)_T$ is the accumulated amount (g/container) of carbon dioxide generated from the composting container containing the test material, $(CO_2)_B$ is the average (g/container) of carbon dioxide accumulated amounts generated from the inoculum source container, and $ThCO_2$ is the theoretical carbon dioxide generation amount confirmed in Equation C above.

**4. Gel strength**

After polymer particles of examples or comparative examples were sieved through a sieve of about 50 mesh, about 0.5 g of the sieved polymer composition was collected. The collected sample was placed in 50 g of a saline solution (0.9 wt% NaCl aqueous solution) and kept at room temperature (about 25°C) for about 1 hour or so to be sufficiently swollen. The unabsorbed saline solution was removed for 4 minutes using an aspirator, and the remaining saline solution was wiped off once using a filter paper. 2.5 g of the swollen sample was placed between two parallel plates with a diameter of 25 mm of a rheometer (TA, ARES-G2), and the gap between the two parallel plates was adjusted to 1 mm. The gap between the parallel plates was adjusted by applying a force of about 3 N so that the swollen sample was in contact with both surfaces of the parallel plates. Subsequently, a shear strain in a linear viscoelastic regime section, where a storage modulus and a loss modulus were constant, was confirmed while increasing the shear strain under an oscillation frequency condition of 10 rad/s. The storage modulus of the sample swollen for 60 seconds in the linear viscoelastic regime section was obtained, and the average of the obtained storage moduli was used as the gel strength. The storage modulus was obtained at room temperature (about 25°C).

**5. Metal component content**

A content of a metal component was measured using an ICP-OES method. First, about 0.05 g of a sample (surface-treated polymer particles of examples or comparative examples) was aliquoted in a Teflon vessel to measure a weight, 0.7 mL of nitric acid was added to the sample, and then a lid was closed and sealed. After dissolving the sample in a 1300 W microwave for 1 hour, it was cooled to room temperature (about 25°C), and then the residue was removed with a filter. An analysis sample was prepared by diluting it with tertiary ultrapure water to 10 mL. The content of the metal

component was measured through the ICP-OES equipment under conditions of RF power 1300 W, torch height 15 mm, plasma gas flow 15 L/min, sample gas flow 0.8 L/min, Aux. gas flow 0.2 L/min, and pump speed 1.5 mL/min.

**6. Measurement of NMR**

A substitution degree of a polysaccharide component was evaluated by performing an NMR analysis on the polysaccharide component. A sample for the NMR analysis was prepared by dissolving 50 mg or so of CMC (carboxymethyl cellulose) in a mixed solvent of 0.75 mL of $D_2O$ and 0.25 mL of $D_2SO_4$, and stirring the mixture in a convection oven maintained at 90°C for 1 hour or so. The $^1H$ NMR analysis of the obtained sample was performed at room temperature (about 25°C) using a Varian Unity Inova (500 MHz) spectrometer having a triple resonance 5 mm probe. The $^1H$ NMR analysis was performed using Bruker's avance Neo instrument.

**7. Evaluation of substitution degree**

A substitution degree of a polysaccharide component was evaluated in the following manner. First, the sum of the integrals of the peaks of 2.57 ppm, 2.58 ppm, 2.60 ppm, 2.64 ppm, 2.66 ppm, 3.15 ppm, 3.16 ppm, 3.33 ppm, and 3.34 ppm, which were peaks within the range of 2.5 ppm to 3.6 ppm of the spectrum measured in the manner described in "6. Measurement of NMR" above, was set to 1. Next, the substitution degree (2-DS) at carbon 2 was obtained by the integral sum at peaks of 3.34 ppm, 3.33 ppm (doublet, 0.23) and 2.57 ppm, and 2.58 ppm (doublet, 0.20), the substitution degree (3-DS) at carbon 3 was obtained by dividing the integral value at peaks of 2.39 ppm, 2.41 ppm (0.20) by 2, and the substitution degree (6-DS) at carbon 6 was obtained by dividing the integral value at peaks of 2.14 ppm, 2.15 ppm (0.19) by 2. Subsequently, the value of 2-DS+3-DS+6-DS was used as the substitution degree by adding all the substitution degrees.

**Example 1.**

CMC (carboxymethyl cellulose) with a substitution degree of about 0.84 or so was used as a polysaccharide component. About 100 g of the CMC and about 0.15 g of a cross-linking agent were dissolved in 4000 mL of distilled water to prepare a mixed solution. $AlCl_3$ was used as the cross-linking agent. The pH of the solution was about 6.5 or so. In a state where the pH of the solution was maintained at about 6.5 or so, the mixed solution was stirred for about 12 hours using a mechanical stirrer at room temperature (25°C) to prepare a polymer solution.

The polymer solution was spread thinly on a tray, and then dried in a convection oven at 40°C for 24 hours to remove the solvent (distilled water). Subsequently, after removing the solvent, the temperature was adjusted to 120°C, and the cross-linking reaction was performed by further maintaining the temperature at the adjusted temperature for about 30 minutes or so.

Subsequently, the obtained cross-linked product was pulverized and classified to obtain polymer particles having a particle size of about 300 $\mu$m to 600 $\mu$m or so.

In the case of these polymer particles, the CRC measured by the above manner was about 69.2 g/g or so, the AUP was about 6.5 g/g or so, and the gel strength was about 115 Pa or so.

The polymer particles were reacted with a surface treatment agent. Maltose oxide was used as the surface treatment agent. The surface treatment agent was mixed with acetone and water in a weight ratio of 0.072:0.6:0.6 (surface treatment agent: acetone: water) to prepare a surface treatment solution. About 3.6 g of the polymer particles was collected and placed on an aluminum dish, and the surface treatment solution was uniformly sprayed on the polymer particles. After the spraying, the solution was evenly mixed with the polymer particles, and then heated at 120°C for 30 minutes to obtain surface-treated polymer particles.

The surface-treated polymer particles had a CRC of about 30.6 g/g or so, an AUP of about 19.6 g/g or so, a gel strength of about 5,236 Pa or so, and a biodegradability degree of about 82% or so, as measured in the above methods.

**Example 2.**

CMC (carboxymethyl cellulose) with a substitution degree of about 0.84 or so was used as a polysaccharide component. About 100 g of the CMC and about 0.3 g of a cross-linking agent ($AlCl_3$) were dissolved in 4,000 mL of distilled water to prepare a mixed solution. The pH of the solution was about 6.5 or so. In a state where the pH of the solution was maintained at about 6.5 or so, the mixed solution was stirred for about 12 hours using a mechanical stirrer at room temperature (25°C) to prepare a polymer solution.

The polymer solution was spread thinly on a tray, and then treated under the same conditions as in Example 1 to remove the solvent (distilled water). After removing the solvent, the temperature was adjusted to 120°C, and the cross-linking reaction was performed by further maintaining the temperature at the adjusted temperature for about 30 minutes or

so. Subsequently, the cross-linked product as cross-linked was pulverized and classified to obtain polymer particles having a particle size of about 300 μm to 600 μm or so.

In the case of these polymer particles, the CRC measured in the above manner was about 60.5 g/g or so, the AUP was about 6.4 g/g or so, and the gel strength was about 221 Pa or so.

The surface treatment was performed on the polymer particles in the same manner as in Example 1. The surface-treated polymer particles had a CRC of about 28.7 g/g or so, an AUP of about 20.6 g/g or so, a gel strength of about 6,179 Pa or so, and a biodegradability degree of about 80% or so, as measured in the above methods.

**Example 3.**

CMC (carboxymethyl cellulose) with a substitution degree of about 0.84 or so was used as a polysaccharide component. About 100 g of the CMC and about 0.5 g of a cross-linking agent ($AlCl_3$) were dissolved in 4000 mL of distilled water to prepare a mixed solution. The pH of the mixed solution was about 6.5 or so. In a state where the pH of the solution was maintained at about 6.5 or so, the mixed solution was stirred for about 12 hours using a mechanical stirrer at room temperature (25°C) to prepare a polymer solution.

The polymer solution was spread thinly on a tray, and then treated under the same conditions as in Example 1 to remove the solvent (distilled water). After removing the solvent, the temperature was adjusted to 120°C, and the cross-linking reaction was performed by further maintaining the temperature at the adjusted temperature for about 30 minutes or so. Subsequently, the cross-linked product as cross-linked was pulverized and classified to obtain polymer particles having a particle size of about 300 μm to 600 μm or so.

In the case of these polymer particles, the CRC measured in the above manner was about 41.7 g/g or so, the AUP was about 6.4 g/g or so, and the gel strength was about 501 Pa or so.

The surface treatment was performed on the polymer particles in the same manner as in Example 1. The surface-treated polymer particles had a CRC of about 23.5 g/g or so, an AUP of about 18.7 g/g or so, a gel strength of about 8,053 Pa or so, and a biodegradability degree of about 81% or so, as measured in the above methods.

**Comparative Example 1.**

CMC (carboxymethyl cellulose) with a substitution degree of about 0.84 or so was used as a polysaccharide component. About 100 g of the CMC and about 0.02 g of a cross-linking agent ($AlCl_3$) were dissolved in 4,000 mL of distilled water to prepare a mixed solution. The pH of the solution was about 6.5 or so. In a state where the pH of the solution was maintained at about 6.5 or so, the mixed solution was stirred for about 12 hours using a mechanical stirrer at room temperature (25°C) to prepare a polymer solution.

The polymer solution was spread thinly on a tray, and then treated under the same conditions as in Example 1 to remove the solvent (distilled water). Subsequently, after removing the solvent, the temperature was adjusted to 120°C, and the cross-linking reaction was performed by further maintaining the temperature at the adjusted temperature for about 30 minutes or so. Subsequently, the obtained cross-linked product was pulverized and classified to obtain polymer particles having a particle size of about 300 μm to 600 μm or so.

In the case of these polymer particles, the CRC measured in the above manner was about 75.1 g/g or so, the AUP was about 5.6 g/g or so, and the gel strength was about 73 Pa or so.

The surface treatment was performed on the polymer particles in the same manner as in Example 1. The surface-treated polymer particles had a CRC of about 29.5 g/g or so, an AUP of about 11.1 g/g or so, a gel strength of about 2,411 Pa or so, and a biodegradability degree of about 75% or so, as measured in the above methods.

**Comparative Example 2.**

CMC (carboxymethyl cellulose) with a substitution degree of about 0.84 or so was used as a polysaccharide component. About 100 g of the CMC and about 1 g of a cross-linking agent ($AlCl_3$) were dissolved in 4,000 mL of distilled water to prepare a mixed solution. The pH of the solution was about 6.5 or so. In a state where the pH of the solution was maintained at about 6.5 or so, the mixed solution was stirred for about 12 hours using a mechanical stirrer at room temperature (25°C) to prepare a polymer solution.

The polymer solution was spread thinly on a tray, and then treated under the same conditions as in Example 1 to remove the solvent (distilled water). Subsequently, after removing the solvent, the temperature was adjusted to 120°C, and the cross-linking reaction was performed by maintaining the temperature at the adjusted temperature for about 30 minutes or so. Subsequently, the cross-linked product as cross-linked was pulverized and classified to obtain polymer particles having a particle size of about 300 μm to 600 μm or so.

In the case of these polymer particles, the CRC measured in the above manner was about 24.5 g/g or so, the AUP was about 7.1 g/g or so, and the gel strength was about 2,412 Pa or so.

The surface treatment was performed on the polymer particles in the same manner as in Example 1. The surface-treated polymer particles had a CRC of about 10.1 g/g or so, an AUP of about *10.5 g/g* or so, a gel strength of about 24,755 Pa or so, and a biodegradability degree of about 65% or so, as measured in the above methods.

**Comparative Example 3.**

CMC (carboxymethyl cellulose) with a substitution degree of about 0.84 or so was used as a polysaccharide component. About 100 g of the CMC and about 0.15 g of a cross-linking agent ($AlCl_3$) were dissolved in 4000 mL of distilled water to prepare a mixed solution. 1 M NaOH was added to the mixed solution to adjust the pH of the solution to about 10.5 or so, and in a state where the pH of the solution was maintained at about 10.5 or so, the mixed solution was stirred for about 12 hours using a mechanical stirrer at room temperature (25°C) to prepare a polymer solution. The polymer solution was spread thinly on a tray, and then treated under the same conditions as in Example 1 to remove the solvent (distilled water). Subsequently, after removing the solvent, the temperature was adjusted to 120°C, and the cross-linking reaction was performed by further maintaining the temperature at the adjusted temperature for about 30 minutes or so. Subsequently, the cross-linked product as cross-linked was pulverized and classified to obtain polymer particles having a particle size of about 300 μm to 600 μm.

In the case of these polymer particles, the CRC measured by the above manner was about 64.2 g/g or so, the AUP was about 6.0 g/g or so, and the gel strength was about 155 Pa or so.

The surface treatment was performed on the polymer particles in the same manner as in Example 1. The surface-treated polymer particles had a CRC of about 32.5 g/g or so, an AUP of about 11.9 g/g or so, a gel strength of about 2,175 Pa or so, and a biodegradability degree of about 75% or so, as measured in the above methods.

**Comparative Example 4.**

CMC (carboxymethyl cellulose) with a substitution degree of about 0.84 or so was used as a polysaccharide component. About 100 g of the CMC and about 0.5 g of a cross-linking agent ($AlCl_3$) were dissolved in 4000 mL of distilled water to prepare a mixed solution. 1 M NaOH was added to the solution to adjust the pH of the solution to about 10.5 or so, and in a state where the pH of the solution was maintained at about 10.5 or so, the mixed solution was stirred for about 12 hours using a mechanical stirrer at room temperature (25°C) to prepare a polymer solution. The polymer solution was spread thinly on a tray, and then treated under the same conditions as in Example 1 to remove the solvent (distilled water). After removing the solvent, the temperature was adjusted to 120°C and the cross-linking reaction was performed by further maintaining the temperature at the adjusted temperature for about 30 minutes or so. Subsequently, the cross-linked product as cross-linked was pulverized and classified to obtain polymer particles having a particle size of about 300 μm to 600 μm.

In the case of these polymer particles, the CRC measured in the above manner was about 47.9 g/g or so, the AUP was about 5.9 g/g or so, and the gel strength was about 296 Pa or so.

The surface treatment was performed on the polymer particles in the same manner as in Example 1. The surface-treated polymer particles had a CRC of about 33.5 g/g or so, an AUP of about 12.1 g/g or so, a gel strength of about 2,311 Pa or so, and a biodegradability degree of about 75% or so, as measured in the above methods.

**Comparative Example 5.**

Carboxymethyl cellulose (CMC) with a substitution degree of about 0.84 or so was used as a polysaccharide component. About 100 g of the CMC and about 0.15 g of a cross-linking agent ($AlCl_3$) were dissolved in 4000 mL of distilled water to prepare a mixed solution. 1 M HCl was added to the solution to adjust the pH of the solution to about 5.5 or so, and in a state where the pH of the solution was maintained at about 5.5 or so, the mixed solution was stirred for about 12 hours using a mechanical stirrer at room temperature (25°C) to prepare a polymer solution. The polymer solution was spread thinly on a tray, and then treated under the same conditions as in Example 1 to remove the solvent (distilled water). Subsequently, after removing the solvent, the temperature was adjusted to 120°C, and the cross-linking reaction was performed by further maintaining the temperature at the adjusted temperature for about 30 minutes or so. Subsequently, the cross-linked product as cross-linked was pulverized and classified to obtain polymer particles having a particle size of about 300 μm to 600 μm.

In the case of these polymer particles, the CRC measured in the above manner was about 14.2 g/g or so, the AUP was about 6.2 g/g or so, and the gel strength was about 2,442 Pa or so.

The surface treatment was performed on the polymer particles in the same manner as in Example 1. The surface-treated polymer particles had a CRC of about 12 g/g or so, an AUP of about *10.1* g/g or so, a gel strength of about 25,887 Pa or so, and a biodegradability degree of about 65% or so, as measured in the above methods.

**Comparative Example 6.**

CMC (carboxymethyl cellulose) with a substitution degree of about 0.84 or so was used as a polysaccharide component. About 100 g of the CMC and about 0.5 g of a cross-linking agent ($AlCl_3$) were dissolved in 4000 mL of distilled water to prepare a mixed solution. 1 M HCl was added to the solution to adjust the pH of the solution to about 5.5 or so, and in a state where the pH of the solution was maintained at about 5.5 or so, the mixed solution was stirred for about 12 hours using a mechanical stirrer at room temperature (25°C) to prepare a polymer solution. The polymer solution was spread thinly on a tray, and then treated under the same conditions as in Example 1 to remove the solvent (distilled water). Subsequently, after removing the solvent, the temperature was adjusted to 120°C, and the cross-linking was performed by further maintaining the temperature at the adjusted temperature for about 30 minutes or so. Subsequently, the cross-linked product as cross-linked was pulverized and classified to obtain polymer particles having a particle size of about 300 μm to 600 μm.

In the case of these polymer particles, the CRC measured in the above manner was about 11.1 g/g or so, the AUP was about 6.1 g/g or so, and the gel strength was about 3,009 Pa or so.

The surface treatment was performed on the polymer particles in the same manner as in Example 1. The surface-treated polymer particles had a CRC of about 13.1 g/g or so, an AUP of about 11.3 g/g or so, a gel strength of about 26,013 Pa or so, and a biodegradability degree of about 65% or so, as measured in the above methods.

**Comparative Example 7.**

CMC (carboxymethyl cellulose) with a substitution degree of about 0.84 or so was used as a polysaccharide component. About 100 g of the CMC and about 0.15 g of a cross-linking agent ($AlCl_3$) were dissolved in 4,000 mL of distilled water to prepare a mixed solution. The pH of the solution was about 6.5 or so. In a state where the pH of the solution was maintained at about 6.5 or so, the mixed solution was stirred for about 12 hours using a mechanical stirrer at room temperature (25°C) to prepare a polymer solution. The polymer solution was maintained in an oven at 70°C for about 40 minutes or so to perform a cross-linking reaction. After the cross-linking reaction, the temperature was adjusted to 40°C and maintained at the adjusted temperature for about 24 hours or so to remove the solvent (distilled water).

Subsequently, the cross-linked product as cross-linked was pulverized and classified to obtain polymer particles having a particle size of about 300 μm to 600 μm.

In the case of these polymer particles, the CRC measured in the above manner was about 67.5 g/g or so, the AUP was about 4.8 g/g or so, and the gel strength was about 65 Pa or so.

The surface treatment was performed on the polymer particles in the same manner as in Example 1. The surface-treated polymer particles had a CRC of about 34.2 g/g or so, an AUP of about 11.6 g/g or so, a gel strength of about 2,387 Pa or so, and a biodegradability degree of about 78% or so, as measured in the above methods.

**Comparative Example 8.**

CMC (carboxymethyl cellulose) with a substitution degree of about 1.27 or so was used as a polysaccharide component. About 100 g of the CMC and about 0.15 g of a cross-linking agent ($AlCl_3$) were dissolved in 4,000 mL of distilled water to prepare a mixed solution. The pH of the solution was about 6.5 or so. In a state where the pH of the solution was maintained at about 6.5 or so, the mixed solution was stirred for about 12 hours using a mechanical stirrer at room temperature (25°C) to prepare a polymer solution. The polymer solution was spread thinly on a tray, and then treated under the same conditions as in Example 1 to remove the solvent (distilled water). Subsequently, after removing the solvent, the temperature was adjusted to 120°C, and the cross-linking reaction was performed by further maintaining the temperature at the adjusted temperature for about 30 minutes or so. Subsequently, the cross-linked product as cross-linked was pulverized and classified to obtain polymer particles having a particle size of about 300 μm to 600 μm.

In the case of these polymer particles, the CRC measured in the above manner was about 61.3 g/g or so, the AUP was about 5.1 g/g or so, and the gel strength was about 81 Pa or so.

The surface treatment was performed on the polymer particles in the same manner as in Example 1. The surface-treated polymer particles had a CRC of about 33.5 g/g or so, an AUP of about 10.8 g/g or so, a gel strength of about 2,451 Pa or so, and a biodegradability degree of about 61% or so, as measured in the above methods.

The physical properties measured for the polymer compositions of Examples and Comparative Examples above were summarized and described in Tables 1 and 2 below. In Tables 1 and 2 below, the metal component content is the content of Al component in the particles after the surface treatment measured in the above-described manner. Also, in Tables 1 and 2 below, BR is physical properties for the polymer particles before the surface treatment, and PD is physical properties for the polymer particles after the surface treatment.

In addition, in Tables 1 and 2 below, the units of CRC and AUP are g/g, and the unit of gel strength is Pa.

[Table 1]

| | | Example | | |
|---|---|---|---|---|
| | | 1 | 2 | 3 |
| Metal component content (Al content) (ppm) | | 166 | 308 | 512 |
| BR | CRC | 69.2 | 60.5 | 41.7 |
| | AUP | 6.5 | 6.4 | 6.4 |
| | Gel strength | 115 | 221 | 501 |
| PD | CRC | 30.6 | 28.7 | 23.5 |
| | AUP | 19.6 | 20.6 | 18.7 |
| | Gel strength (Pa) | 5236 | 6179 | 8053 |
| | Biodegradability degree (%) | 82 | 80 | 81 |

[Table 2]

| | | Comparative Example | | | | | | | |
|---|---|---|---|---|---|---|---|---|---|
| | | 1 | 2 | 3 | 4 | 5 | 6 | 7 | 8 |
| Metal component content (Al content) (ppm) | | 23 | 1016 | 156 | 520 | 159 | 519 | 154 | 158 |
| BR | CRC | 75.1 | 24.5 | 64.2 | 47.9 | 14.2 | 11.1 | 67.5 | 61.3 |
| | AUP | 5.6 | 7.1 | 6 | 5.9 | 6.2 | 6.1 | 4.8 | 5.1 |
| | Gel strength | 73 | 2412 | *155* | 296 | 2442 | 3009 | 65 | 81 |
| PD | CRC | 29.5 | 10.1 | 32.5 | 33.5 | 12 | 13.1 | 34.2 | 33.5 |
| | AUP | 11.1 | 10.5 | 11.9 | 12.1 | 10.1 | 11.3 | 11.6 | 10.8 |
| | Gel strength | 2411 | 24755 | 2175 | 2311 | 25887 | 26013 | 2387 | 2451 |
| | Biodegradability degree (%) | 75 | 65 | 75 | 75 | 65 | 65 | 78 | 61 |

From the results in Tables 1 and 2, it can be confirmed that the polymer compositions of Examples exhibit excellent absorption capacity (particularly, absorption capacity under pressure) together with appropriate gel strength and biodegradability capacity. This shows that the purpose can be achieved by adjusting the amount of the metal compound and the cross-linking process in the cross-linking process using the metal compound.

For example, in Comparative Example 1, the cross-linking process was performed using the same materials and method as in an example, but the amount of the cross-linking agent (metal compound) was small, so that it exhibited the AUP value and gel strength lower than those of the example. In addition, when the gel strength is low, the CRC and biodegradability degree are secured relatively well, but in the case of Comparative Example 1, the CRC and biodegradability degree were also poor relative to the example.

In Comparative Example 2, the cross-linking process was performed using the same materials and method as in an example, but the amount of the cross-linking agent (metal compound) was large, so that the absorption characteristics and biodegradability degree were significantly lowered relative to the example, and it exhibited excessively high gel strength.

In Comparative Examples 3 and 4, the cross-linking process was performed using the same materials and method as in an example, but the pH was adjusted high upon the cross-linking process. As a result, they exhibited the AUP values and gel strengths lower than those of the example, and when the gel strength is low, the biodegradability degree is secured relatively well, but in the case of Comparative Example 3, the biodegradability degree was also poor relative to the example.

In Comparative Examples 5 and 6, the cross-linking process was performed using the same materials and method as in an example, but the pH was controlled low upon the cross-linking process, and as a result, the absorption characteristics and biodegradability degree were significantly lowered relative to the example, and they exhibited excessively high gel strength.

In Comparative Example 7, the cross-linking process was performed using the same materials and method as in

an example, but the solvent removal process was not performed before the cross-linking process, and the removal of the solvent was performed after the cross-linking process, but as a result, it exhibited the AUP value and gel strength lower than those of the example. Normally, when the gel strength is low, the biodegradability degree is secured relatively well, but in the case of Comparative Example 7, the biodegradability degree was also poor relative to the example.

In Comparative Example 8, the cross-linking process was performed using the same materials and method as in an example, but the CMC with a high substitution degree was used, and as a result, it exhibited the AUP value and gel strength lower than those of the example. Normally, when the gel strength is low, the biodegradability degree is secured relatively well, but in the case of Comparative Example 7, the biodegradability degree was also poor relative to the example.

The reason why the pH appears similar at about 6.5 or so despite the different contents of $AlCl_3$, a weakly acidic salt, in the mixed solutions of Examples 1 to 3 and Comparative Examples 1 and 2 is because in the mixed solutions, the excessive amount of CMC is contained, and the very small amount of $AlCl_3$ compared to the CMC is contained, so that the pH of the solutions is dominantly determined by the CMC, and the pH effect by the $AlCl_3$ is not significant.

## Claims

1. A polymer composition comprising

    a polysaccharide component and a metal component, and
    having an absorption capacity under pressure (AUP) at 0.7 psi of 13 g/g or more according to EDANA method WSP 242.3, and
    having a gel strength of 2,500 Pa or more.

2. The polymer composition according to claim 1, wherein the polysaccharide component has a content of 85 wt% or more.

3. The polymer composition according to claim 1, wherein the metal component has a content in inductively coupled plasma optical emission spectrometry (ICP-OES) in a range of 100 to 1,000 ppm.

4. The polymer composition according to claim 1, having a centrifuge retention capacity (CRC) of 15 g/g or more according to EDANA method WSP 241.3.

5. The polymer composition according to claim 1, having a biodegradability degree of 75% or more.

6. The polymer composition according to claim 1, wherein the metal component is derived from an aluminum compound.

7. The polymer composition according to claim 6, wherein the aluminum compound is one or more selected from the group consisting of aluminum acetate, aluminum sulfate, aluminum chloride, and aluminum lactate.

8. The polymer composition according to claim 1, being in the form of a polymer particle including a core, and a surface layer including a compound covalently bonded to the surface of the core,
wherein the core comprises the polysaccharide component and the metal component.

9. The polymer composition according to claim 8, wherein the polysaccharide component and the metal component in the core are coordinately bonded to each other.

10. The polymer composition according to claim 1, wherein the polysaccharide component is an acidic polysaccharide component having a substitution degree in a range of 0.1 to 2.5.

11. The polymer composition according to claim 1, wherein the polysaccharide component contains a unit represented by Formula 1 below:

[Formula 1]

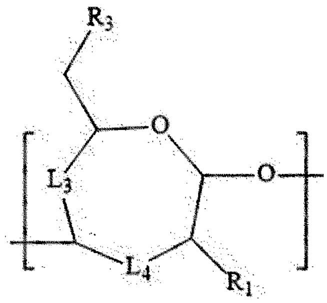

wherein, $R_1$ is a hydroxy group, an amino group, $-L_5-C(=O)-OH$, $-L_5-C(=O)-O-$, or a functional group of Formula 2 below, $R_3$ is a hydroxy group, $-L_5-C(=O)-OH$, $-L_5-C(=O)-O-$, or a functional group of Formula 2 below, and any one of $L_3$ and $L_4$ is a single bond, and the other is $CHR_2$, where $R_2$ is a hydroxy group, $-L_5-C(=O)-OH$, $-L_5-C(=O)-O-$, or a functional group of Formula 2 below, and $L_5$ is an alkylene group or an alkylidene group, provided that an oxygen or nitrogen atom of any one of $R_1$ to $R_3$ above forms a bond with the metal component:

[Formula 2]

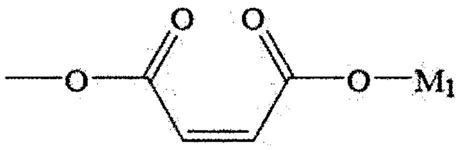

wherein, $M_1$ is hydrogen or a metal, and when $M_1$ is the metal, the bond of $O-M_1$ is an ionic bond.

**12.** The polymer composition according to claim 8, wherein the compound is one or more selected from the group consisting of a polyfunctional epoxy compound, an epoxy silane compound, an amino silane compound, epichlorohydrin, an acyl chloride, a carbonate, a dialdehyde, a diamine, a diol, carbon disulfide, phosphoryl chloride, divinyl benzene, an organic acid, and an organic acid anhydride.

**13.** The polymer composition according to claim 8, wherein the compound is a disaccharide having two or more aldehyde groups or carboxyl groups.

**14.** A method for preparing the polymer composition of any one of claims 1 to 13, comprising a step of cross-linking, in a polymer solution containing a polysaccharide component, a metal compound, and a solvent, the polysaccharide component with the metal compound.

**15.** The method for preparing the polymer composition according to claim 14, wherein the pH of the polymer solution is maintained in a range of 6 to 10 upon cross-linking.

**16.** The method for preparing the polymer composition according to claim 14, wherein the metal compound in the polymer solution is present at a ratio of 0.01 to 10 parts by weight relative to 100 parts by weight of the polysaccharide component.

**17.** The method for preparing the polymer composition according to claim 14, comprising a first step of maintaining the polymer solution at a temperature T1; and a second step of maintaining the polymer solution at a temperature T2, wherein

the temperature T1 is adjusted so that $\Delta T1$ of Equation 1 below is in a range of 10°C to 90°C, and
the temperature T2 is adjusted so that $\Delta T2$ of Equation 2 below is in a range of 5°C to 200°C:

[Equation 1]

$$\Delta T1 = Tb - T1$$

[Equation 2]

$$\Delta T2 = T2 - Tb$$

wherein, Tb is the boiling point of the solvent of the polymer solution under 1 atm, T1 is the temperature T1 of the first step, and T2 is the temperature T2 of the second step.

**18.** The method for preparing the polymer composition according to claim 17, wherein the temperature T1 is in a range of 30°C to 200°C, and the first step is performed for 10 hours to 60 hours.

**19.** The method for preparing the polymer composition according to claim 17, wherein the temperature T2 is in a range of 80°C to 300°C, and the second step is performed for 10 minutes to 10 hours.

**20.** The method for preparing the polymer composition according to claim 14, comprising a step of granulating a cross-linked product after cross-linking; and a step of reacting the granulated cross-linked product with a compound.

## INTERNATIONAL SEARCH REPORT

International application No.
**PCT/KR2024/017525**

**A. CLASSIFICATION OF SUBJECT MATTER**

**C08K 5/098**(2006.01)i; **C08K 5/1545**(2006.01)i; **C08K 3/30**(2006.01)i; **C08L 1/28**(2006.01)i; **C08L 5/00**(2006.01)i; **C08B 15/00**(2006.01)i; **C08B 37/00**(2006.01)i; **C08J 3/12**(2006.01)i; **A61L 15/60**(2006.01)i

According to International Patent Classification (IPC) or to both national classification and IPC

**B. FIELDS SEARCHED**

Minimum documentation searched (classification system followed by classification symbols)

C08K 5/098(2006.01); A61F 13/15(2006.01); A61F 13/53(2006.01); A61L 15/22(2006.01); C08B 11/12(2006.01); C08B 15/00(2006.01); C08B 15/04(2006.01); C08J 3/24(2006.01); C08L 1/08(2006.01); C08L 1/10(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal), STN (Registry, Caplus), Google & keywords: 하이드로겔 폴리머(hydrogel polymer), 다당류(polysaccharide), 카르복시메틸 셀룰로오스(CMC: carboxymethyl cellulose), 금속 성분 가교제(AlCl3), 가압 하 흡수능(AUP: absorption under pressure), 원심분리 보수능(CRC: centrifuge retention capacity)

**C. DOCUMENTS CONSIDERED TO BE RELEVANT**

| Category* | Citation of document, with indication, where appropriate, of the relevant passages | Relevant to claim No. |
|---|---|---|
| X | KR 10-0914096 B1 (WEYERHAEUSER COMPANY) 27 August 2009 (2009-08-27)<br>See claims 1 and 5; paragraphs [0010], [0014] and [0021]; and table 1. | 1-7,10,11,14,16 |
| Y | | 8,9,12,13,15,17-20 |
| Y | KR 10-2019-0080165 A (LOTTE FINE CHEMICAL CO., LTD.) 08 July 2019 (2019-07-08)<br>See claims 1, 3, 4 and 6; and paragraphs [0027], [0063], [0065] and [0075]-[0078]. | 8,9,12,13,15,17-20 |
| A | KR 10-2006-0076243 A (WEYERHAEUSER COMPANY) 04 July 2006 (2006-07-04)<br>See claims 8 and 15; and paragraphs [0036], [0042], [0048] and [0058]. | 1-20 |
| A | KR 10-1508405 B1 (KIMBERLY-CLARK WORLDWIDE, INC.) 06 April 2015 (2015-04-06)<br>See claims 1 and 9; and paragraph [0171]. | 1-20 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **20 February 2025** | **20 February 2025** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

INTERNATIONAL SEARCH REPORT

International application No.
PCT/KR2024/017525

C. DOCUMENTS CONSIDERED TO BE RELEVANT

| Category* | Citation of document, with indication, where appropriate, of the relevant passages | Relevant to claim No. |
|---|---|---|
| A | JP 2005-502735 A (STOCKHAUSEN GMBH AND CO KG) 27 January 2005 (2005-01-27) See claim 15; and paragraphs [0036], [0037], [0039], [0128] and [0130]. | 1-20 |

Form PCT/ISA/210 (second sheet) (July 2022)

**INTERNATIONAL SEARCH REPORT**
**Information on patent family members**

International application No.
**PCT/KR2024/017525**

| Patent document cited in search report | Publication date (day/month/year) | Patent family member(s) | Publication date (day/month/year) |
|---|---|---|---|
| KR 10-0914096 B1 | 27 August 2009 | CA 2603610 A1 | 02 April 2008 |
| | | CA 2603610 C | 17 August 2010 |
| | | CN 101164625 A | 23 April 2008 |
| | | CN 101164625 B | 13 June 2012 |
| | | EP 1911466 A1 | 16 April 2008 |
| | | EP 1911466 B1 | 21 October 2009 |
| | | KR 10-2008-0030948 A | 07 April 2008 |
| | | MX 2007012273 A | 28 October 2008 |
| | | US 2008-0081191 A1 | 03 April 2008 |
| | | US 2008-0081843 A1 | 03 April 2008 |
| | | US 7645806 B2 | 12 January 2010 |
| | | US 7785710 B2 | 31 August 2010 |
| KR 10-2019-0080165 A | 08 July 2019 | KR 10-2600854 B1 | 10 November 2023 |
| KR 10-2006-0076243 A | 04 July 2006 | CA 2529199 A1 | 29 June 2006 |
| | | CA 2529199 C | 08 December 2009 |
| | | CN 1796654 A | 05 July 2006 |
| | | EP 1676864 A1 | 05 July 2006 |
| | | EP 1676864 B1 | 08 March 2017 |
| | | JP 2006-188698 A | 20 July 2006 |
| | | MX PA05013773 A | 28 June 2006 |
| | | US 2006-0137838 A1 | 29 June 2006 |
| | | US 7541396 B2 | 02 June 2009 |
| KR 10-1508405 B1 | 06 April 2015 | AU 2008-313369 A1 | 23 April 2009 |
| | | AU 2008-313369 B2 | 04 July 2013 |
| | | BR PI0816529 A2 | 23 April 2009 |
| | | BR PI0816529 B1 | 21 November 2018 |
| | | BR PI0816529 B8 | 22 June 2021 |
| | | CN 101827616 A | 08 September 2010 |
| | | CN 101827616 B | 30 April 2014 |
| | | EP 2207576 A2 | 21 July 2010 |
| | | EP 2207576 A4 | 31 October 2012 |
| | | EP 2207576 B1 | 15 June 2016 |
| | | JP 2011-500124 A | 06 January 2011 |
| | | JP 5260663 B2 | 14 August 2013 |
| | | MX 2010004037 A | 30 April 2010 |
| | | US 2009-0099541 A1 | 16 April 2009 |
| | | US 8039683 B2 | 18 October 2011 |
| | | WO 2009-050613 A2 | 23 April 2009 |
| | | WO 2009-050613 A3 | 15 October 2009 |
| JP 2005-502735 A | 27 January 2005 | AU 2002-310741 A1 | 09 December 2002 |
| | | BR 0210005 A | 13 April 2004 |
| | | BR 0210005 B1 | 08 October 2013 |
| | | CA 2448140 A1 | 05 December 2002 |
| | | CA 2448140 C | 29 December 2009 |
| | | CN 100471874 C | 25 March 2009 |
| | | CN 1520425 A | 11 August 2004 |
| | | DE 10125599 A1 | 28 November 2002 |
| | | EP 1404719 A1 | 07 April 2004 |
| | | EP 1404719 B1 | 27 February 2019 |
| | | JP 4286129 B2 | 24 June 2009 |

Form PCT/ISA/210 (patent family annex) (July 2022)

**INTERNATIONAL SEARCH REPORT**
**Information on patent family members**

International application No.
**PCT/KR2024/017525**

| Patent document cited in search report | Publication date (day/month/year) | Patent family member(s) | Publication date (day/month/year) |
|---|---|---|---|
| | | TW I304407 B | 21 December 2008 |
| | | US 2004-0157734 A1 | 12 August 2004 |
| | | US 2008-0262155 A1 | 23 October 2008 |
| | | US 7407912 B2 | 05 August 2008 |
| | | US 7612016 B2 | 03 November 2009 |
| | | WO 02-096953 A1 | 05 December 2002 |

Form PCT/ISA/210 (patent family annex) (July 2022)